# EUROPEAN PATENT SPECIFICATION

(11) **EP 1 362 801 B1**
(45) Date of publication and mention of the grant of the patent: **24.01.2007**
(21) Application number: 03252060.3
(22) Date of filing: 01.04.2003
(51) Int. Cl.: B65D 83/08

(54) **Test strip containers and methods of using the same**
Behälter für Teststreifen und Methoden zu deren Verwendung
Récipient pour bandelettes de test et leurs méthodes d'utilisation

(30) Priority: 02.04.2002 US 115834
(43) Date of publication of application: 19.11.2003
(73) Proprietor: LifeScan, Inc., Milpitas, CA 95035-6312 (US)
(72) Inventor: Pugh, Jerry, Mountain View, California 94043 (US)
(74) Representative: Mercer, Christopher Paul

(56) References cited:
- DE-U- 29 905 561

## Description

### FIELD OF THE INVENTION

The field of this invention is test strip containers.

### BACKGROUND OF THE INVENTION

Analyte concentration determination in physiological samples is of ever increasing importance to today's society. Such assays find use in a variety of application settings, including clinical laboratory testing, home testing, etc., where the results of such testing play a prominent role in the diagnosis and management of a variety of disease conditions. Analytes of interest include glucose for diabetes management, cholesterol for monitoring cardiovascular conditions, and the like. In response to this growing importance of analyte concentration determination, a variety of analyte concentration determination protocols and devices for both clinical and home testing have been developed. Many of such analyte concentration determination protocols employ test strip devices.

However, before testing can begin, an individual seeking to determine the presence and/or concentration of an analyte in a physiological sample must first obtain a test strip, apply a sample thereto and obtain the results either manually or automatically with a meter or the like. However, obtaining a test strip to begin the procedure is not without difficulty. The ability to easily obtain a test strip, particularly a single test strip from amongst a plurality of test strips, is important particularly when the containers and test strips will be used by persons with diminished vision and/or hand-eye coordination and/or finger sensation. For example, persons with diabetes typically have one or all of impaired vision, diminished hand-eye coordination and diminished finger sensation or other dexterity problems. Such persons must use test strips to test their blood glucose levels a number of times a day. However, the typical test strip is only several millimeters in width and length and, thus, difficult to grasp and manipulate.

The simplest test strip containers are simple storage reservoirs where the test strips are retained inside and manually removed therefrom. However, it is difficult to easily extract a test strip from these containers. These containers are usually shaped and sized to hold a plurality of test strips and to completely encompass the test strips inside so as to protect the test strips from humidity, etc., where such protection is necessary to insure the precision, accuracy and overall integrity of the test result.

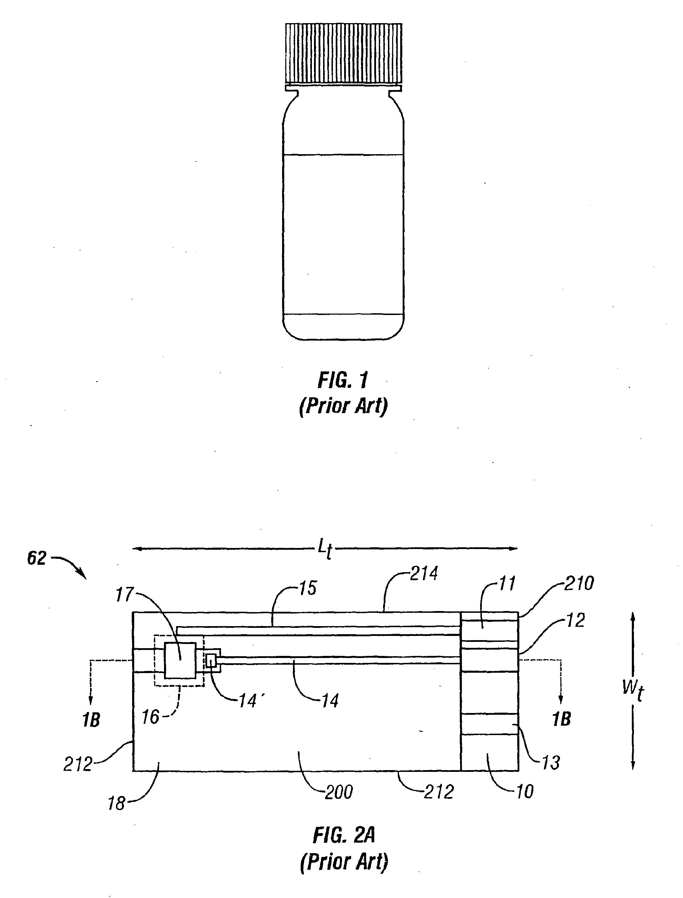

An exemplary embodiment of such a simple test strip container known in the art is shown in Figure 1. To obtain a single test strip from this type of container to begin a test, an individual has two options for removing a test strip. In one option, the cap is removed and an individual may simply turn the container upside down to pour a test strip out. This, as is apparent, has significant disadvantages as one or all of the test strips stored inside the container may quickly spill out and become contaminated or otherwise damaged. In a second option, the cap is removed and an individual places a finger inside the container to try to grasp a single test strip amongst a plurality of test strips. However, such a method is difficult for individuals who have impaired vision and/or diminished finger sensation, etc., as described above. Furthermore, the container must have an opening shaped and sized large enough to accommodate at least one finger therein, for easy removal of a test strip. In other words, the container must enable an individual, e.g., an individual who may be visually and/or dextrally impaired, to grasp a test strip from amongst a plurality of test strips.

It can be appreciated that the container, while maintaining a size large enough to serve its functions, must be small enough to enable portability of the container so that an individual may easily carry the container at all times to accommodate testing during the course of a day. However, due to the above described shape and size requirements, conventional containers are typically cylindrical, i.e., have a circular cross-sectional shape, to accommodate insertion of at least one finger therein, have a height of about 60 mm and a diameter of about 25 mm and are commercially sold with about 25 test strips retained therein. As is apparent, such size and shape creates a great amount of unused space inside the container, minimizes the portability of the container and adds to the container's costs. In other words, the containers are larger than necessary to simply hold the test strips, thus increasing costs and decreasing portability.

More complex test strip containers have been developed to try to overcome some of the disadvantages associated with the simple test strip containers described above (see for example U.S. Patent Nos. 5,575,403, 5,489,414; 5,630,986; 5,510,266). However, these too have certain disadvantages. For example, these devices often require a degree of physical dexterity and visual acuity that may be lacking in certain individuals who use the containers. Also, due to the complexity of the devices, i.e., the number of components forming the containers, the cost of manufacture increases and thus the cost to the user increases. Still further, many of the more complex conventional containers require that the test strips therein be stacked in an orderly or precise manner which also adds to the manufacturing process thereby increasing the costs associated therewith.

As such, there is continued interest in the development of new devices and methods for use in test strip containing and dispensing. Of particular interest would be the development of such devices and methods which are easy and inexpensive to manufacture, have minimal components, are easy to use, particularly for visually and dextrally impaired individuals and are easily portable.

### SUMMARY OF THE INVENTION

Devices for containing at least one test strip and dispensing a single test strip at a time and methods of using the same are provided. The subject devices are characterized by having a housing that includes a slit configured to be permissive of a single test strip at a time and at least one planar surface to align a test strip with the slit when the device is appropriately agitated. In the subject methods, a subject device with at least one test strip therein is provided and agitated in a manner that causes the at least one test strip to be positioned in an appropriate orientation relative to the slit, wherein a single test strip is caused to exit the housing through the slit as a result of the agitation. Also provided are kits that include at least one subject device.

### BRIEF DESCRIPTION OF THE DRAWINGS

Figure 1 shows an exemplary embodiment of a conventional test strip container.
Figure 2A-2D show an exemplary representative electrochemical test strip suitable for use with the subject invention.
Figure 3A shows an exemplary embodiment of a subject device wherein the housing of the device has a substantially annular cross sectional shape. Figure 3B shows another exemplary embodiment of a subject device wherein the housing of the device has a rectangular cross-sectional shape. Figure 3C shows a view taken along lines C-C of Figure 3A.
Figure 4 shows a subject device having a separable cap.
Figure 5 shows an exemplary embodiment of a planar surface that is a surface of a separate component configured to be insertable into a subject device.
Figure 6A shows a perspective view of an exemplary embodiment of a subject housing. Figure 6B shows a view taken along lines A-A of Figure 6A.
Figure 7 shows a perspective view of an exemplary embodiment of a subject housing having a wall that slopes towards the slit of the device.
Figure 8A shows an exemplary embodiment of a subject housing wherein the test strip stop upon which a test strip may rest when it is dispensed from the housing is in a second or closed position to seal the housing. Figure 8B shows the device of Figure 8A wherein the test strip stop is in a first or open position to unseal the housing.
Figure 9A shows an exemplary embodiment of a test strip stop according to the subject invention. Figure 9B shows a schematic side view of the test strip stop of Figure 9A in a closed position. Figure 9C shows a schematic side view of the test strip stop of Figure 9B between the closed and open positions. Figure 9D shows a schematic side view of the test strip stop of Figure 9A in an open position.
Figure 10A shows another exemplary embodiment of a test strip stop according to the subject invention. Figure 10B shows an exploded view of the test strip stop of Figure 10A and a portion of an exemplary housing. Figure 10C shows the configured test strip stop and housing of Figures 10A-10B. Figure 10D shows the device of Figure 10C wherein the test strip stop is in an open position. Figure 10E shows the device of Figure 10D wherein the test strip stop is in a closed position.
Figure 11A shows an exemplary embodiment of a subject device positioned such that the slit of the device is substantially facing towards the ground. Figure 11B shows an individual moving the test strip stop of the device of Figure 11A so that a test strip may be dispensed from the housing.
Figures 12A-12E illustrates the steps of an embodiment of the subject methods wherein a subject device is agitated in a rotational or angular or pendular manner to dispense a test strip therefrom. Figure 12F illustrates the steps of an embodiment of the subject methods wherein a subject device is agitated in a lateral or translational manner to dispense a test strip therefrom.
Figure 13 shows a cut-away, top view into the interior of a subject housing having a plurality of test strips contained therein, wherein the housing is being agitated in a direction that is substantially perpendicular to the longitudinal axis of the slit of the housing.
Figure 14 shows a single test strip being dispensed or exiting from a subject housing through a slit in the housing wherein the exiting test strip has come to rest on the test strip stop of the housing.
Figure 15 shows a view taken along lines B-B of Figure 14.
Figure 16 shows a dispensed test strip being removed from a subject housing.
Figure 17A shows an exemplary embodiment of a subject housing having a slideable test strip stop positioned in a first or open position. Figure 17B shows the housing of Figure 17A with the slideable test strip stop positioned in a second or closed position.

### DETAILED DESCRIPTION OF THE INVENTION

Before the present invention is described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It must be noted that as used herein and in the appended claims, the singular forms "a", "and", and "the" include plural referents unless the context clearly dictates otherwise. Thus, for example, reference to "a reagent" includes a plurality of such reagents and reference to "the device" includes reference to one or more devices and equivalents thereof known to those skilled in the art, and so forth.

In further describing the subject invention, the subject devices are described first. Next, a description of the subject methods is provided, followed by a review of kits which include at least one subject device.

### DEVICES

As summarized above, devices are provided for containing and dispensing test strips. Particularly, devices are provided for containing one or more test strips and easily dispensing a single test strip at a time therefrom and, usually, the subject devices provide for the easy dispensation of a single test strip at a time from amongst a plurality of test strips, i.e., dispenses each test strip separately.

The invention is suitable for dispensing any type of test strip, for example electrochemical and colorimetric or photometric (i.e., optical) type test strips as are known in the art, where such test strips find use in the determination of a wide variety of different analyte concentrations and where representative analytes include, but are not limited to, glucose, cholesterol, lactate, alcohol, and the like. In many embodiments, the test strips used with the subject invention are used to determine the glucose concentration in a physiological sample, e.g., interstitial fluid, blood, blood fractions, constituents thereof, and the like. In further describing the subject invention, a review of representative electrochemical test strips that may find use with the subject invention is provided first to provide a proper foundation for the subject invention, where such a review is by way of example and is not intended to limit the scope of the invention. In other words, it will be apparent that a wide variety of test strips, including, but not limited to, a variety of colorimetric and electrochemical-type test strips, are suitable for use with the present invention. The review of exemplary representative electrochemical test strips is followed by a description of the subject test strip container devices.

### Representative Electrochemical Test Strips

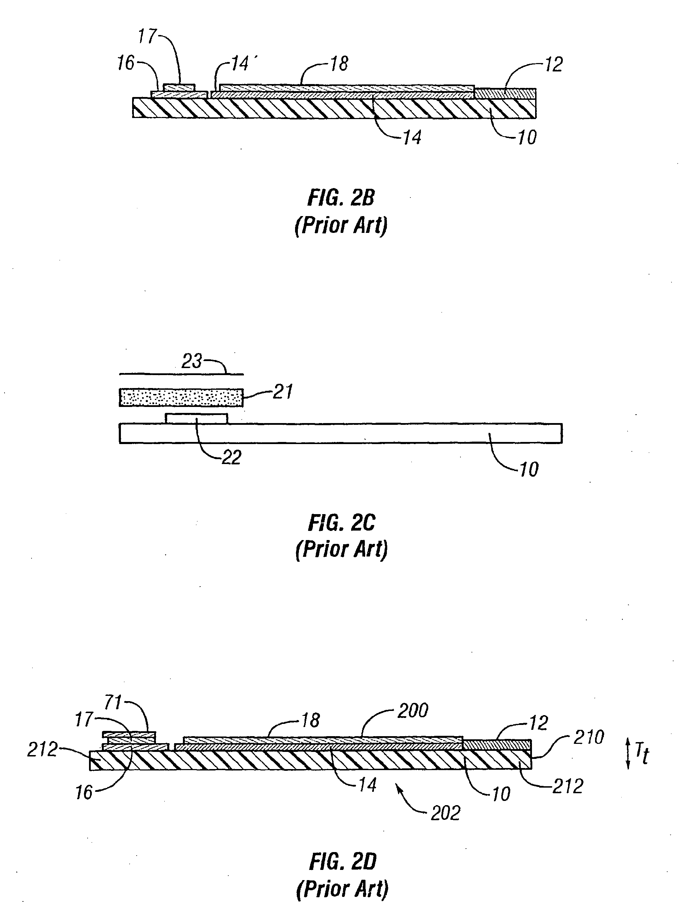

Generally, the electrochemical test strips that find use with the subject invention are made up of two electrodes associated with a substrate. In many embodiments a redox reagent system is located in a reaction area or zone of the test strip. The electrochemical test strips may be configured and adapted to be received in an automated meter, as described below, for automatically determining the concentration of an analyte. Figure 2A shows a planar view of representative test strip 62.

As shown, the electrodes are formed on a substrate 10. On substrate 10 are placed two conductive elements 14' and 16, connected by leads 14 and 15 to conductive contacts 11, 12, and 13. An insulating mask 18 is formed, leaving at least a portion of conductive elements 14' and 16, and contacts 11, 12 and 13 exposed. A non-conductive integrated reagent/blood separation layer 17 is applied over insulating mask 18 to make contact with conductive element 16. Advantageously, test strip 62 is finished by applying a nylon or polyester mesh 21 over the sample application region defined by the location of the integrated reagent/blood separation layer 17 of the electrode assembly 22, and then a top cover 23 to prevent splashing of the blood sample (See Figure 2C). The polyester mesh acts to guide the sample to the reference electrode, conductive element 14'.

The substrate 10 used in making the test strips of the invention can be any nonconducting, dimensionally stable material suitable for insertion into a meter. Suitable materials include polyester films, for example a 330 micron polyester film,and other insulating substrate materials such as polyvinyl chloride (PVC), polyethylene terephthalate (PET), polyethylene terephthalate glycol modified (PETG), polyimide, polystyrene, polycarbonate, silicon, glass and the like.

The conductive elements and associated leads and contacts can be formed from essentially any conductive material including silver, Ag/AgCl, gold, palladium, iridium, stainless steel, platinum, doped tin oxide, carbon, etc., and need not all be formed from the same material. Conductive element 16 may be formed from conductive carbon in certain embodiments. Usually conductive carbon are ERCON ERC1, ERCON ERC2 and Acheson Carbon Electrodag 423. The conductive element 16 makes contact with working electrode track 15, and is close to, but not contacting conductive element 14' disposed as the end of reference electrode track 14.

The insulating layer 18 can be formed from polyester-based printable dielectric materials such as ERCON R488-B(HV)-B2 Blue. The top cover 23 is suitably formed from a polyester strip or a "hot melt" coated plastic.

The integrated reagent/blood separation layer 17 may be formed from a mixture containing a filler which has both hydrophobic and hydrophilic surface regions, and in the case of a glucose test strip, usually an enzyme which can oxidize glucose, and a mediator which can transfer electrons from the enzyme to the underlying conductive element layer 16. This layer is suitably formed by formulating an ink which contains the filler, the enzyme and the mediator in a suitable carrier and using this ink to print the layer 17 onto the device. A typical filler for use in layer 17 is silica ,e.g., silica having a surface modification to render it partially hydrophobic is used. Materials of this type include Cab-O-Sil TS610, a silica which is modified by partial surface treatment with methyl dichlorosilane; Cab-o-Sil 530, a silica which is modified by full surface treatment with hexamethyl disilazane; Spherisorb C4 silica, which is surface modified with 4 carbon chains; and other similarly modified silicas, or combinations thereof. Integrated reagent/blood separation layer 17 is usually formed from an aqueous composition containing a binder such as hydroxyethylcellulose or mixtures of hydroxyethylcellulose with alginate or other thickeners, silica; and a redox reagent system or composition, as mentioned above, where the reagent system interacts with components in the fluid sample during the assay.

The redox reagent system present typically includes at least an enzyme(s) and a mediator. In many embodiments, the enzyme member(s) of the redox reagent system is an enzyme or plurality of enzymes that work in concert to oxidize the analyte of interest. In other words, the enzyme component of the redox reagent system is made up of a single analyte oxidizing enzyme or a collection of two or more enzymes that work in concert to oxidize the analyte of interest. Enzymes of interest include oxidases, dehydrogenases, lipases, kinases, diaphorases, quinoproteins and the like.

The specific enzyme present in the reaction area depends on the particular analyte for which the electrochemical test strip is designed to detect, where representative enzymes include: glucose oxidase, glucose dehydrogenase, cholesterol esterase, cholesterol oxidase, lipoprotein lipase, glycerol kinase, glycerol-3-phosphate oxidase, lactate oxidase, lactate dehydrogenase, pyruvate oxidase, alcohol oxidase, bilirubin oxidase, uricase, and the like. In many preferred embodiments where the analyte of interest is glucose, the enzyme component of the redox reagent system is a glucose oxidizing enzyme, e.g. a glucose oxidase or glucose dehydrogenase.

The second component of the redox reagent system is a mediator component, which is made up of one or more mediator agents. A variety of different mediator agents are known in the art and include: ferricyanide, phenazine ethosulphate, phenazine methosulfate, pheylenediamine, 1-methoxy-phenazine methosulfate, 2,6-dimethyl-1,4-benzoquinone, 2,5-dichloro-1,4-benzoquinone, ferrocene derivatives, osmium bipyridyl complexes, ruthenium complexes and the like. In those embodiments where glucose in the analyte of interest and glucose oxidase or glucose dehydrogenase are the enzyme components, mediator of particular interest is ferricyanide. Other reagents that may be present include buffering agents, e.g. citraconate, citrate, phosphate, "Good" buffers, antifoam and the like. Yet other agents that may be present include: divalent cations such as calcium chloride, and magnesium chloride; surfactants such as Triton, Macol, Tetronic, Silwet, Zonyl, and Pluronic; stabilizing agents such as albumin, sucrose, trehalose, mannitol, and lactose.

Generally test strip 62 can be characterized as having two broad surfaces or sides - first broad surface 200 and second broad surface 202 that is opposite broad surface 200, and edges 210, 212, 214 and 216 (see Figures 1A and 1D). As depicted, test strip 62 is generally configured in the form of an elongate strip. Typically, the length Lₜ of test strip 62, ranges from about 0. 5 to 2 in (1.3 to 5.1 cm), usually from about 0.79 to 1.1 in (2.0 to 2.8 cm). The width Wₜ of test strip 62 typically ranges from about 0.15 to 0.50 in (0.38 to 1.3 cm), usually from about 0.20 to 0.27 in (0.51 to 0.67 cm). The total thickness of test strip 62 Tₜ typically ranges from about 0.010 inch (about 250 µm) to about 0.040 inch (about 1000 µm), usually from about 0.02 inch (about 500 µm) to about 0.03 inch (about 750 µm)

Examples of such a reagent test strips suitable for use with the subject invention include those described in U.S. Patent No. 5,708,247; 6,241,862; 6,193,873, and WO 01/67099, as well as in EP-A-1 252 514, EP-A-1 254 365, WO-A-2/48707 and WO-A-02/50609.

To use such an electrochemical test strip, an aqueous liquid sample (*e.g*., blood) is placed into the sample application region. The amount of physiological sample that is introduced into the region of the test strip may vary, but generally ranges from about 0.1 to 10 µl, usually from about 0.3 to 0.6 µl. The sample may be introduced into the reaction area using any convenient protocol, where the sample may be injected into the reaction area, allowed to wick, or be otherwise introduced through the ports.

The component to be analyzed is allowed to react with the redox reagent coating to form an oxidizable (or reducible) substance in an amount corresponding to the concentration of the component to be analyzed (i.e., analyte). The quantity of the oxidizable (or reducible) substance present is then estimated by an electrochemical measurement.

The measurement that is made may vary depending on the particular nature of the assay and the device with which the electrochemical test strip is employed (e.g., depending on whether the assay is coulometric, amperometric or potentiometric). Measurement with test strip 62 is usually accomplished by way of an automated instrument or meter. Usually, measurement is taken over a given period of time following sample introduction to the test strip. Methods for making electrochemical measurements are further described in U.S. Patent Nos.: 4,224,125; 4,545,382; and 5,266,179; as well as WO 97/18465 and WO 99/49307 publications.

Following detection of the electrochemical signal, as described above, the amount of the analyte present in the sample introduced into the test strip is then typically determined by relating the electrochemical signal to the amount of analyte in the sample. In making this derivation, the electrochemical signal is usually compared to the signal generated from a series of previously obtained control or standard values, and determined from this comparison. In many embodiments, the electrochemical signal measurement steps and analyte concentration derivation steps are performed automatically by a device designed to work with the test strip to produce a value of analyte concentration in a sample applied to the test strip, as noted above. A representative reading device for automatically practicing these steps, such that user need only apply sample to the reaction zone and then read the final analyte concentration result from the device, is further described in WO 01/64105 and U.S. Patent Nos. 6,193,873 and 5,266,179.

### Test Strip Containers

As described above, the subject invention includes test strip container devices that are configured to contain and easily dispense a single test strip at a time such as the type of test strip described above, where the subject test strip containers have minimal components, don't require precise or orderly stacking of the test strips therein, are portable, are easy to use and are easy and inexpensive to manufacture. Typically, the subject devices dispense a single test strip at a time from a plurality or aggregate of contained test strips. Usually the subject devices are configured to retain from about 1 to about 50 test strips at one time, usually about 5 to about 40 test strips at one time and more usually from about 10 to about 25 test strips at one time, however the subject devices may be configured to retain a greater or fewer number of test strips at one time. The subject devices may also be configured to allow for easy re-loading of additional test strips, as will be apparent from the descriptions below.

A feature of the subject devices is that they do not require a user to activate the device or any component thereof in order for the device to dispense a single test strip for use. Rather, the devices are passively activated in that they need only be shaken or agitated or the like in order for a single test strip to be dispensed therefrom and thus are particularly well suited for use by an individual having visual and/or dexterity problems such as reduced hand-eye coordination, reduced finger sensation, etc., as will be apparent upon reading this disclosure.

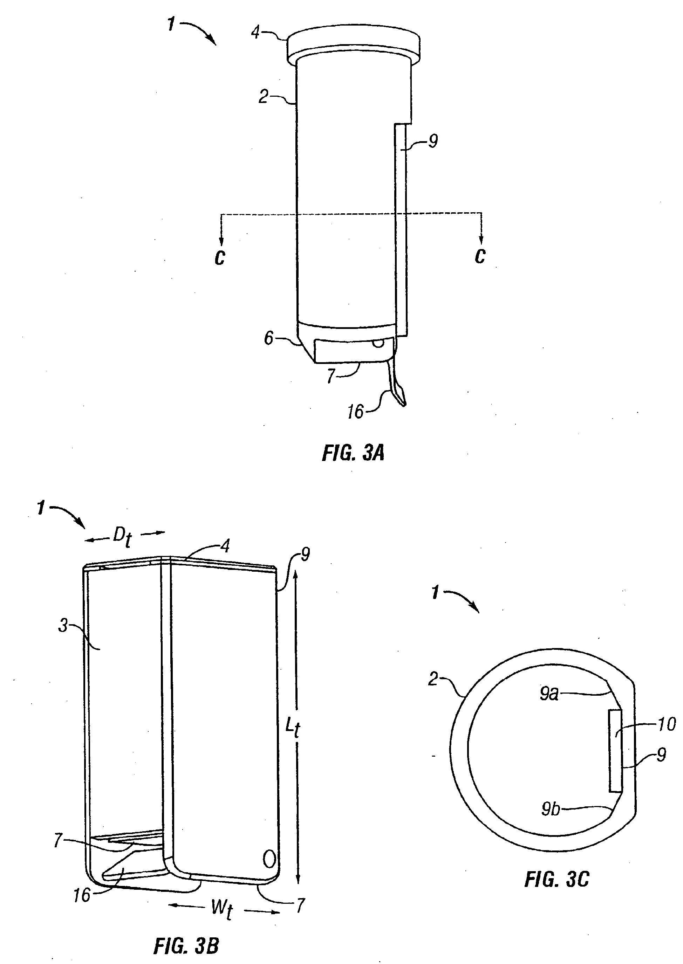

The subject devices will now be described with reference to the Figures, where like numerals represent like components or features. Figure 3A shows an exemplary embodiment of a subject test strip container device 1 that includes housing 2 having first end 4 and second end 6 enclosing a space therebetween and having a substantially annular shape. As shown, housing 2 includes at least one planar surface 9 facing the interior of housing 2 (shown in Figure 3A as a view from the exterior however, in all embodiments of the subject invention, the interior of the housing has at least one planar surface) and test strip stop 16, both of which will be described in greater detail below.

The shape of the subject device will necessarily vary depending on a variety of factors, where such factors include, but are not limited to, the type, size and number of test strips retained therein, and the like. Generally, device 1 is shaped to be easily and comfortably held by and individual. Figure 3A shows housing 2 having a cross sectional shape that is substantially annular, but other cross sectional shapes are possible as well. That is, the cross sectional shape of the housing may have any of a variety of shapes ranging from simple to complex. For example, the cross sectional shape of the housing may be substantially circular, elliptical, oval, rectangular, square, etc., where a rectangular cross sectional shape is of particular interest. Figure 3B shows an exemplary embodiment of a housing 3 having a rectangular cross sectional shape. Alternatively, as mentioned, the shape of the housing may be more complex such as a substantially irregular shape or the like. In further describing the subject invention, housing 3 will oftentimes be used as a representative housing. However, it is to be understood that this is by way of example and not limitation. That is, in all embodiments of the subject invention, the housing may have any of a variety of cross-sectional shapes as described above, e.g., annular as shown in Figure 3A.

Likewise, the size of the housing may also vary depending on a variety of factors such as the type, size and number of test strips retained therein, and the like. The housing will typically be sized to be easily and comfortably held and transported by an individual. By way of example only and not limitation, in those embodiments where the housing contains one or more test strips, each test strip having a length ranging from about 19 mm to about 51 mm, a width ranging from about 3.8 mm to about 7.6 mm and a thickness ranging from about 0.3 mm to about 1.0 mm and the housing has a substantially rectangular shape (see Figure 3B), the length L_{H} of the housing, i.e., the height, typically ranges from about 30 mm to about 60 mm, usually from about 40 mm to about 55 mm and the width W_{H} of housing 3 may range from about 15 mm to about 35 mm, usually from about 15 mm to about 25 mm and the diameter D_{H} i.e., the thickness, typically ranges from about 10 mm to about 30 mm, usually from about 15 mm to about 20 mm.

The housing may be manufactured from a variety of materials, where such materials will not substantially interfere with the testing reagents of the test strips retained therein. Representative materials that may be used in the fabrication of the housing include, but are not limited to, polymeric materials such as polytetrafluoroethylene, polypropylene, polyethylene, polystyrene, polycarbonate, polysulphone, and blends thereof, metals such as stainless steel, aluminum and alloys thereof, TEFLON™, other fluorocarbons, siliceous material, *e.g*., glass materials, and the like.

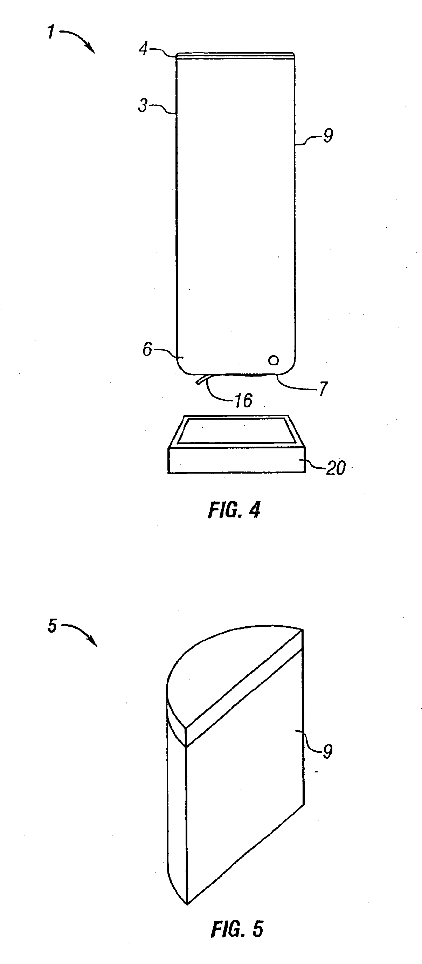

Any of the above described embodiments may include one or more sealing elements such as one or more caps or lids for sealing one or both of first end 4 and second end 6, if they are open to the outside environment on all or a portion thereof, to provide a substantially moisture-tight interior housing environment. As shown in Figure 4 in regards to housing 3, housing 3 includes optional cap 20 configured to seal second end 6. In other embodiments a cap may be used to seal first end 4 in addition to or in place of sealing second end 6.

Cap 20 may be associated with the subject housing using any convenient means, such that cap 20 may be a threaded cap which may be screwed onto the end of the housing, cap 20 may snap fit together with the housing, cap 20 may be hinged to the housing, e.g., on one side, cap 20 may be frictionally associate with the housing, and the like. Cap 20 may be manufactured from a wide variety of materials, including those materials described above with respect to the housing. A gasket (not shown) may be positioned between cap 20 and the housing to provide a substantially moisture tight seal therebetween so as to maintain the integrity of the test strip(s) contained inside the housing.

A feature of the subject invention is that the subject housing includes at least one planar surface 9 facing the interior thereof, (in other words the at least one planar surface is within the housing) as previously described (see for example Figures 3A, 3B and 3C, where Figure 3C shows a view taken along lines C-C of Figure 3A and shows the at least one planar surface facing the interior of the housing). The at least one planar surface 9 may be a surface of a separate component 5 that is inserted into the interior of the housing (see for example Figure 5 which is configured to be inserted inside housing 2), may be integrally formed with the housing such as injected molded or the like or may be a portion of the housing itself, such as a portion of a wall of the housing, as shown in Figures 3A, 3B and 3C. In those embodiments where the at least one planar surface 9 is provided by a component that is not integrally formed with the housing, the materials from which it is fabricated include those described above as representative materials suitable for the fabrication of the housing. Such a separate component is typically configured to be snugly fit within the housing such that the shape thereof usually, although not always, corresponds to the inner surface(s) of the housing (see for example Figure 5 which shows a planar surface component 5 that is configured to conform to the inner surface of housing 2) and such that the planar surface component 5 will not substantially move within the housing upon shaking or agitation of the housing. The planar surface component 5 may be held in a fixed position in the interior of the housing using any suitable means, including, but not limited to, adhesives, friction, snap fit, ledges or ridges, etc.

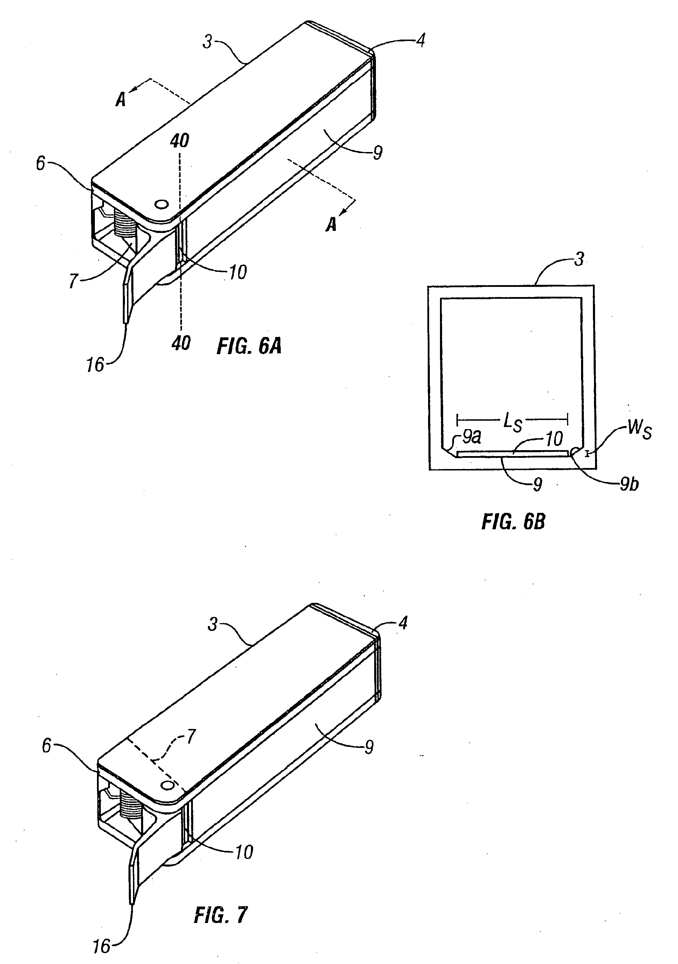

The dimensions of the at least one planar surface 9 will vary depending on the dimensions of the housing, the dimensions of the slit of the housing, the test strip(s) contained therein, etc. Typically, the length of the at least one planar surface 9 ranges from at least about 50% or more of the length of the test strip(s) contained in the housing, where in certain embodiments the length of the at least one planar surface 9 ranges from about 75% or more of the length of the test strip(s) contained in the housing. The width of the at least one planar surface 9 is usually at least as wide as the length of the slit of the housing. That is, in many embodiments, the width of the least one planar surface 9 is usually substantially the same as the length of slit 10. As such, the width of the at least one planar surface 9 is at least as wide as the width of a test strip contained in the housing and is usually greater than the width of such a test strip such that the width of the at least one planar surface is usually at least about 125% to about 195% of the width of a test strip contained in the device, usually at least about 150% to about 180% of the width of a test strip. In certain embodiments, the at least one planar surface has beveled edges, see for example beveled edges 9a and 9b of housing 2 of Figure 3C and of housing 3 in Figure 6B.

As mentioned above, ultimately a single test strip is aligned with and enters a slit positioned in the housing, where the slit provides an opening from the exterior of the housing to the interior. That is, the slit opening transverses the entire thickness of a wall or floor of the housing, e.g., bottom wall 7 of housing 2 and housing 3. In using the subject device to dispense a test strip, a test strip is caused to enter and be positioned in the slit. Usually a portion of the exited test strip resides outside the housing and a portion, usually a substantial portion or greater than about 50% of the length of the exited test strip resides outside the housing and the remaining portion of the test strip remains inside the housing, as will be described in greater detail below.

Figure 6A shows a perspective view of housing 3. As shown, slit 10 is positioned in bottom wall 7 of second end 6, adjacent at least one planar surface 9 such that the longitudinal axis 40 of slit 10 is substantially parallel to the at least one planar surface 9, i.e., slit 10 and at least one planar surface are substantially aligned, e.g., are coplanar. Figure 6B shows a view taken along lines A-A of Figure 6A. As shown in Figure 6B, slit 10 and the at least one planar surface 9 are aligned such that they share a common wall, i.e., there is no space, or substantially little space, between the at least one planar surface 9 and slit 10. In certain embodiments, bottom wall 7 of second end 6 may be somewhat sloped downward towards slit 10 so as to facilitate directing the test strips contained towards slit 10, as shown in Figure 7.

The slit of the subject invention is sized and shaped for accommodating or being permissive of only a single test strip therein at one time. However, the size and/or shape of the slit need not necessarily correspond to that of a test strip, i.e., the size and/or shape of the slit may differ from the size and/or shape (width) of a test strip, as long as the slit enables only a single test strip to be inserted therein at one time, e.g., a test strip may be rectangular in shape and the slit may be of a shape other than rectangular. Accordingly, the width Wₛ (see Figure 6B) of the slit is greater than the thickness of a single test strip contained in the housing, but less than the thickness of two test strips. Likewise, the length Lₛ of the slit (see Figure 6B) is greater than the width of a single test strip contained in the housing, but less than the width of two test strips. For example, the width Wₛ of slit 10 is usually about 125% to about 195% of the thickness Tₜ of a test strip and the length Lₛ of slit 10 is usually about 125% to about 195% of the width Wₜ of a test strip.

The subject invention also includes a test strip stop associated with the slit of the housing. The test strip stop is configured to at least prevent a test strip from completely exiting the housing. That is, as mentioned above, usually a substantial portion or greater than about 50% of the length of the test strip is positioned outside the housing and the remaining portion of the test strip is positioned inside the housing. In other words, the test strip stop is appropriately positioned in a first position relative to the slit of the housing such that a test strip may exit the housing via the slit in the housing, but the partially exited test strip is prevented from completely exiting the housing or exiting the housing entirely such that the partially exited test strip is instead caught or held by the test strip stop. Figure 6A shows test strip stop 16 positioned adjacent to slit 10 in a first position so that a test strip exiting housing 3 of device 1 through slit 10 will come to rest on test strip stop 16 and thus cannot completely exit the housing.

The test strip stop may be moveable or immoveable, where a moveable test strip stop may also be configured to seal the housing or rather the slit of the housing when the test strip stop is positioned in a certain manner, such that a test strip is completely prevented or blocked from exiting the housing through the slit of the housing, i.e., the housing is sealed to the outside environment so that a test strip is prevented from even partially protruding from the slit of the housing because the slit is sealed.

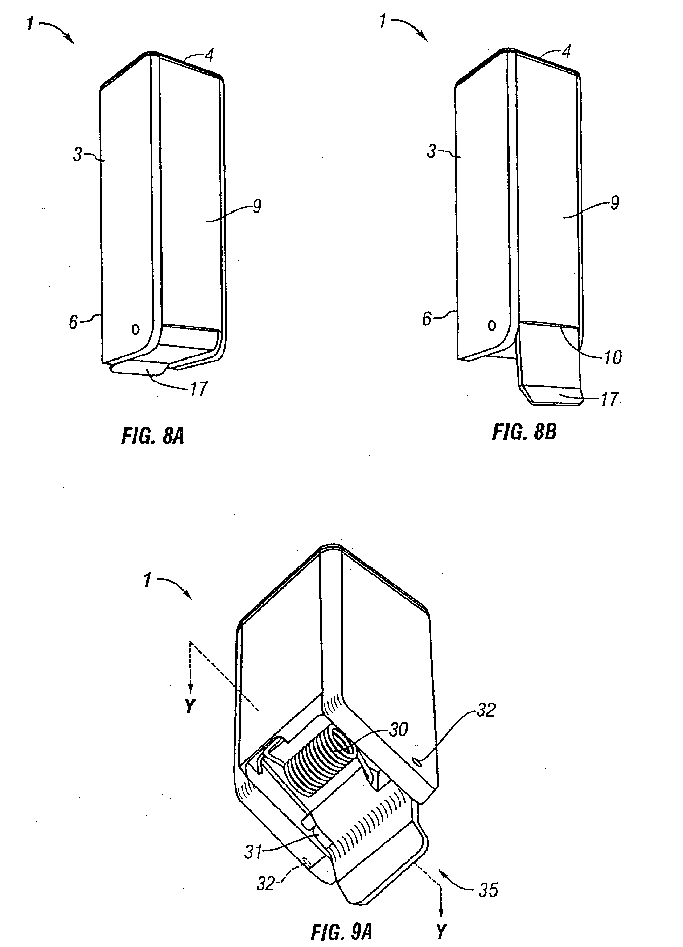

As described above, the test strip stop may be moveable. In one such embodiment, the test strip stop is moveable from a first or down position that unseals or opens the housing and allows communication between the interior of the housing and the external environment via the slit in the housing to a second or up position that seals or closes the housing, i.e., seals the slit of the housing. One such embodiment is shown in Figures 8A and 8B. More specifically, Figure 8A shows a perspective view of housing 3 of device 1 having moveable test strip stop 17 in a second position such that slit 10 of housing 3 is sealed, i.e., the interior of housing 3 is not in communication with the outside environment and no test strips can be dispensed therefrom. When moved or toggled to a first position as shown in Figure 8B, test strip stop 17 exposes slit 10 such that slit 10 provides an opening between the interior of the housing 3 and the outside environment, whereby a test strip may be dispensed. In this first position, test strip stop 17 is positioned to catch a test strip that exits the housing 3 through slit 10 and prevents it from completely exiting the housing.

The test strip stop of the subject invention may be configured in any convenient manner to enable it to move between a first position and a second position, where the following embodiments are provided by way of example and are in no way intended to limit the scope of the invention.

One embodiment is shown in Figures 9A to 9D. Figure 9A shows a perspective, bottom view of housing 3 of device 1 having moveable test strip stop 35 in a first or down position such that housing 3 is unsealed. Test strip stop 35 is configured to move or toggle between the first and second positions. In this embodiment, moveable test strip stop 35 is associated with torsion spring 30 and pivot pins 32, where pins 32 are positionable in holes in the bottom surface of the housing to provide pivot points. Housing 3 also includes stop 31 positioned to enable test strip stop 35 to assume an appropriate position to hold an exiting test strip.

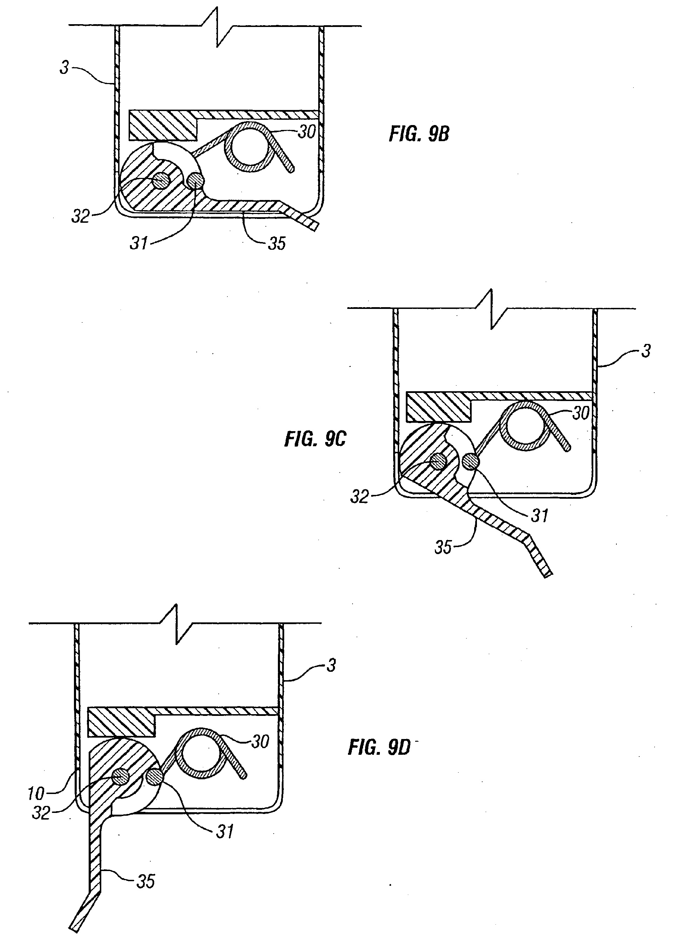

Figures 9B to 9D schematically show the manner by which torsion spring 30 enables test strip stop 35 to toggle between the first and second positions. Figure 9B shows a view taken along lines Y-Y of Figure 9A and shows test strip stop 35 in a second, closed or up position such that slit 10 is sealed by test strip stop 35. In this sealed position, torsion spring 30 produces a counterclockwise torque on test strip stop 35, holding it in the second position. As shown in Figure 9C, test strip stop 35 moves to a first, open or down position by moving clockwise around pivot point 32. At this stage, no torque is produced on test strip stop 35. In the first or open position shown in Figure 9D whereby slit 10 is exposed or unsealed, torsion spring 30 produces a clockwise torque on test strip stop 35 to hold test strip stop 35 in this first position against stop 31 so that a test strip exiting via slot 10 will come to rest on test strip stop 35 so as not to completely exit the housing.

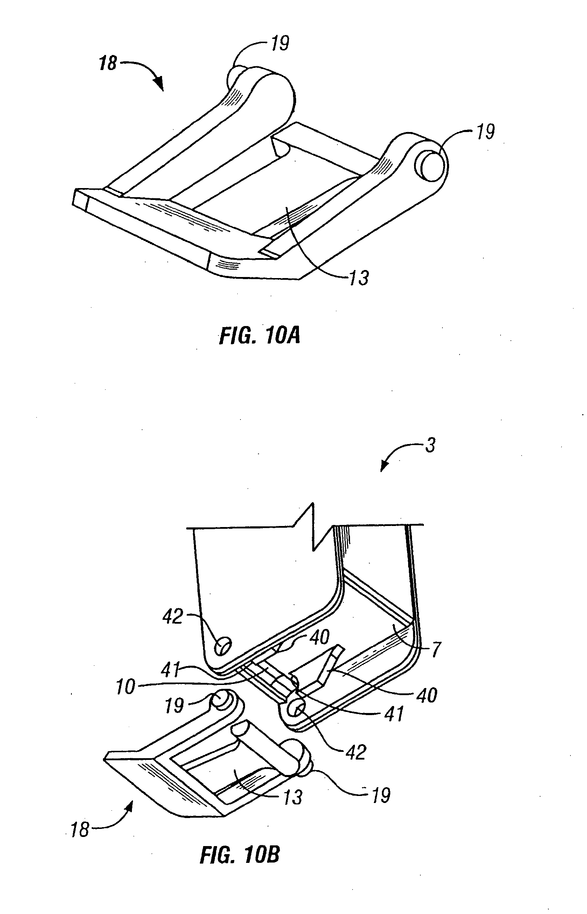

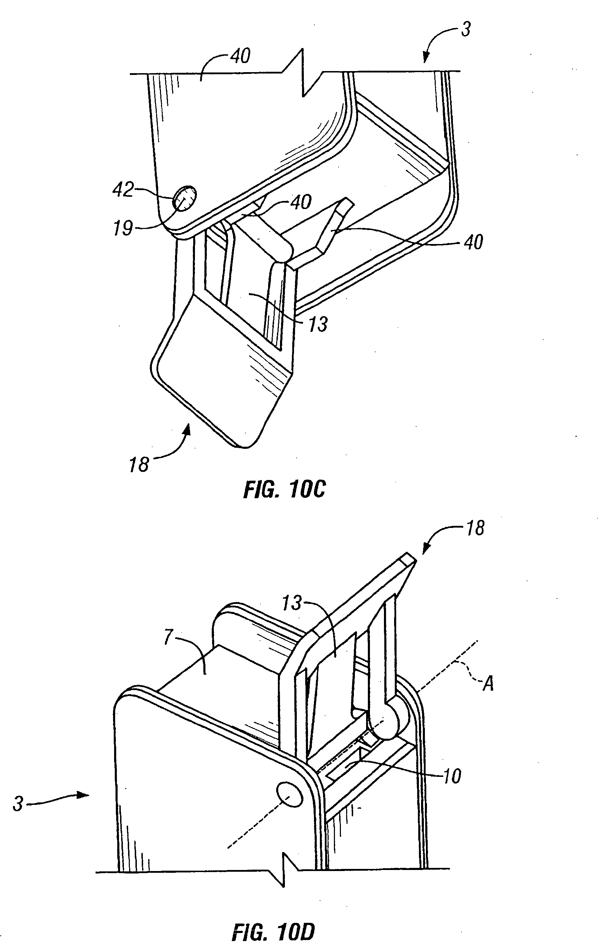

In another embodiment, the moveable test strip stop does not include a torsion spring. Figure 10A shows an enlarged view of moveable test strip stop 18 which includes pivot pins 19 and central leaf 13. Figure 10B shows an exploded view of test strip stop 18 and a housing, herein shown for exemplary purposes as housing 3, but it will be apparent that test strip stop 18 may be used with any housing embodiment such as housing 3 and the like. Figure 10C shows test strip stop 18 and housing 3 associated or assembled together such that pivot pins 19 are positioned in holes 42 to provide pivot points and test strip stop 18 is in the first, unsealed or open position. As shown, on bottom wall 7 of housing 3 is positioned stops 40 having notches 42.

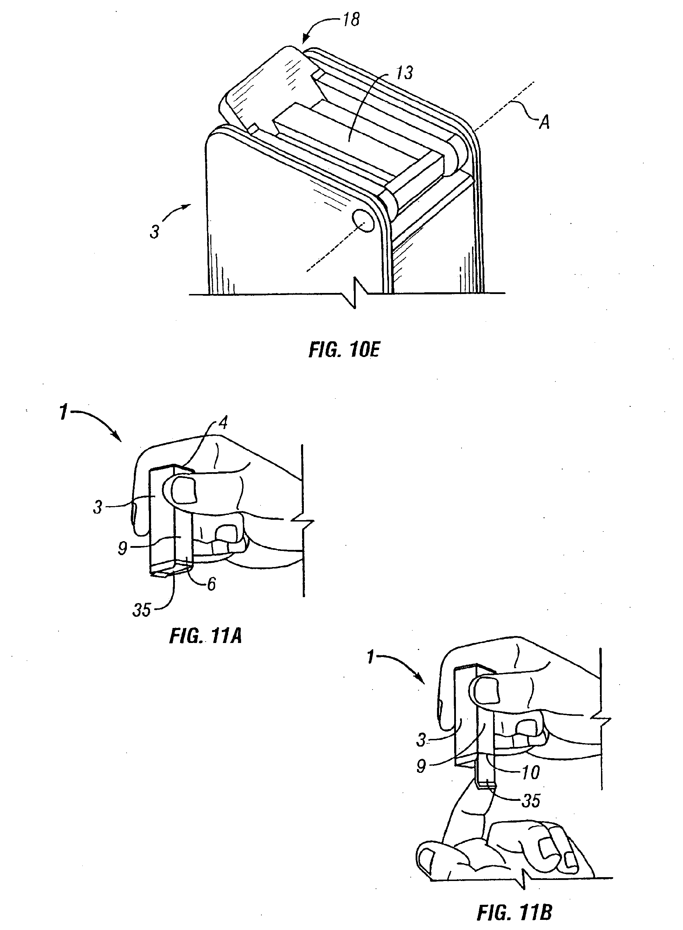

Referring now to Figures 10D and 10E, it is shown that in the first or open position shown in Figure 10D, whereby slit 10 is exposed, test strip stop 18 is partially constrained about axis A due to a portion of central leaf 13 abutting stops 40. In other words, test strip stop 18 and stops 40 are configured to enable test strip stop 18 to move from a second or closed position shown in Figure 10E about 90° to a first position shown in Figure 10D, where it is stopped from further movement, i.e., beyond about 90°, by stops 40. In the first position, central leaf 13 engages notches 41, thereby holding test strip stop 18 in the first position so that a test strip exiting the housing through the slit of the housing will come to rest on test strip stop 18 and thus cannot completely exit the housing. The second or closed position is shown in Figure 10E. In such a closed position, central leaf 18 seats over slit 10 to provide a substantially moisture tight seal over slit 10.

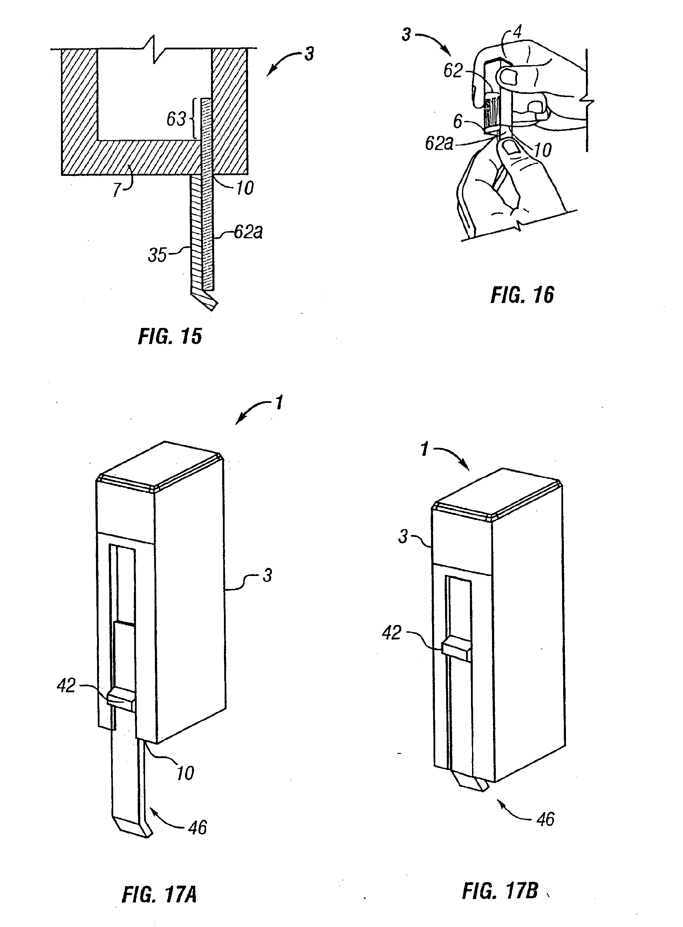

In another embodiment shown in Figures 17A and 17B, the test strip stop is a slideable member 46 that is configured to move or slide along a portion of the housing between a first position that unseals or opens the housing as shown in Figure 17A to a second position that seals or closes the slit of the housing as shown in Figure 17B. Test strip stop 46 usually includes a finger tab 42 for engagement by an individual, where tab 42 may be knurled to optimize gripping by an individual's finger.

All embodiments of the subject housing may further include moisture absorbent reagents or components such as desiccant material, silica gel and the like, where such material is capable of absorbing moisture from the environment surrounding the stored test strips. Such absorbent reagents or components may be retained in one or more compartments positioned in a convenient location inside the housing.

### SYSTEMS

The above described devices may find use with systems that include the devices of the subject invention and at least one of an automated device or meter for determining the concentration of an analyte in a sample and one or more test strips, such as the type of test strips described above. Such meters are well known in the field of analyte concentration determination.

### METHODS

Also provided by the subject invention are methods for containing at least one test strip and dispensing a single test strip at a time. More specifically, methods are provided that enable a single test strip to be easily dispensed from a container that houses the test strip so that the test strip may be used, for example by a visually and/or dextrally impaired individual. According to the subject methods, a subject test strip container device as described above is provided and at least one test strip is provided therein. The device is agitated in an appropriate manner, which causes the at least one strip to become positioned in a suitable orientation relative to the direction or plane of agitation and thus to the slit in the device. Once at least one test strip is appropriately oriented relative to the slit, a single test strip enters the slit and thus is dispensed for use. Usually a plurality of test strips is contained in the device and thus only one test strip is dispensed at a time from the device from amongst a plurality of test strips such that the remainder of the test strips continues to be held in the device for use at a later time. Accordingly, the subject methods advantageously enable a single test strip to be dispensed for use by simply agitating or shaking the subject device in a certain manner, where such methods are particularly well suited for visually and/or dextrally impaired individuals.

As such, the first step in the subject methods is to provide a subject test strip container, where such a container is configured to store at least one test strip and more likely a plurality of test strips therein, as described above. In further describing the subject methods, housing 3 having moveable test strip stop 35 containing a plurality of test strips 62 therein is used for exemplary purposes only and is in no way intended to limit the scope of the invention. That is, the description of the subject methods is applicable to all embodiments of the subject invention and combinations thereof, unless otherwise noted. For example the subject methods are applicable to all embodiments of the subject device, such as all housing embodiments, e.g., housing 3, all embodiments of the at least one planar surface, as well as all embodiments of the test strip stop, whether moveable or immoveable, and any combinations thereof, where all embodiments of the subject device may contain one test strip or a plurality of test strips.

Once the provision of a suitable test strip container is met, at least one test strip, such as at least one test strip of the type described above, is provided therein, where the at least one test strip may be preloaded in the housing, e.g., at the point of manufacture, or may be loaded in the housing by an individual after the point of manufacture. (In certain embodiments, loading one or more test strips in the housing after the point of manufacture may also require adding more or new moisture absorbing material such as desiccant or the like to the housing as well.) The at least one test strip is positioned within the housing on one of its edges, e.g., edges 210 or 212 of test strip 62. In many embodiments, the at least one test strip is positioned in a manner whereby the sample application portion of the test strip(s) is closest to the bottom wall than the opposite end of the test strip(s) is closest to the bottom wall so that the sample application portion is the first portion of a test strip to exit from the housing. The at least one test strip may, in certain embodiments, also be positioned such that the sample application portion of the test strip(s) is not obscured by the test strip stop when the test strip is held or caught by the test strip stop. In this manner, sample may be applied to an exited test strip while the test strip remains held by the test strip stop. Advantageously, unlike conventional test strip containers (see for example U.S. Patent No. 4,911,344), the test strips need not be stacked in an orderly or precise manner in the housing as long as each test strip is appropriately positioned in the housing, i.e., each test strip is positioned on one of its edges, as noted above. As is apparent, eliminating the requirement to stack the test strips eliminates steps in the manufacturing process and thus reduces costs.

As shown in Figure 11A, housing 3of device 1 is held by an individual so that slit 10 is substantially facing towards the ground G, i.e., second end 6 is closer to the ground G than first end 4 is closer to the ground G. Housing 3, if sealed, is opened to dispense a test strip. In the embodiment shown in Figure 11A, the test strip stop is a moveable test strip stop and thus is moved from the second position that seals the housing as shown in Figure 11A to a first position that unseals the housing and provides a catch for an exiting test strip as shown in Figure 11B. Specifically, as shown in Figure 11A, moveable test strip stop 35 is in a second position such that housing 3 is sealed or closed, i.e., slit 10 is not exposed. To dispense a test strip, housing 3 is opened to the outside environment as shown in Figure 11B, by moving or rotating (sliding as in the case of a slideable test strip stop) moveable test strip stop 35 to a first position such that slit 10 is exposed or open so that a test strip may pass from the interior of housing 3 to the exterior of housing 3 via slit 10 and test strip stop 35 is positioned to catch a test strip exiting housing 3 through slit 10. Likewise, if other sealing means are present which prevent a test strip from being dispensed from the housing such as a cap, the cap is separated from the housing.

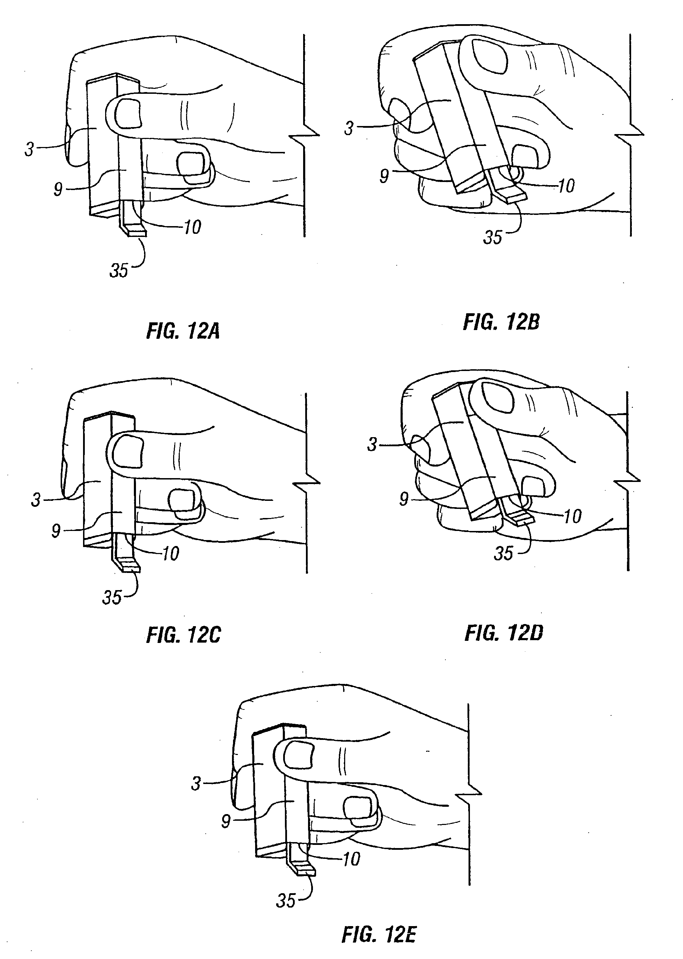

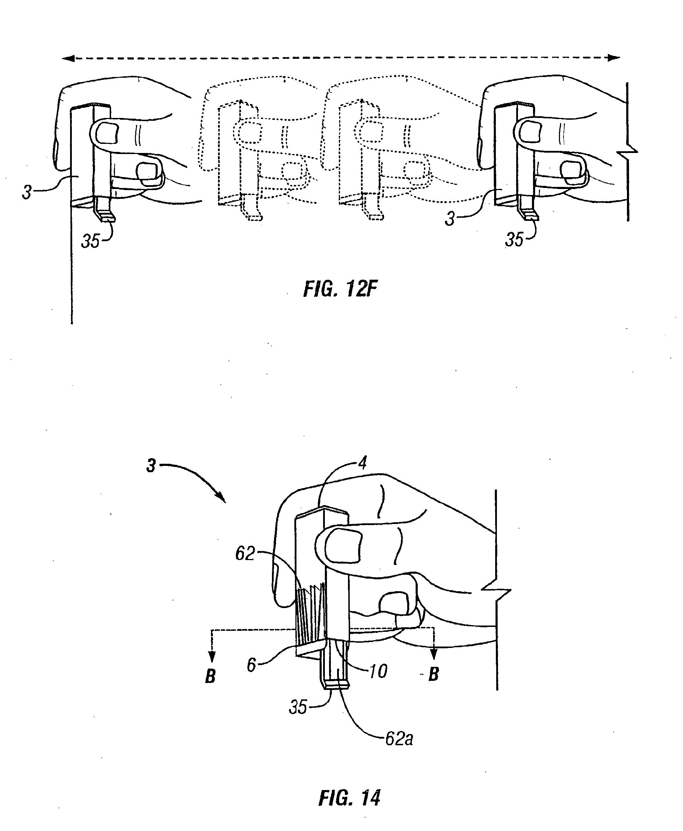

The housing is then agitated or shaken to position or align the at least one test strip therein in an appropriate orientation relative to slit 10 using at least one planar surface 9. Housing 3 may be agitated or moved in any convenient manner to accomplish the above-described agitation and orientation, as long as the general direction of the movement is substantially perpendicular to the longitudinal axis of slit 10. For example, as illustrated in Figures 12A-12E, housing 3 is agitated in a rotational, angular or pendular manner. In another embodiment, housing 3 is agitated in a lateral or translational manner as illustrated in Figure 12F. Of course, the agitation of the housing may be accomplished in any suitable manner.

Regardless of the manner in which the housing is agitated, the housing is agitated an appropriate number of times, i.e., one or more times, until a test strip is caused to enter slit 10, where the exact number of times the housing is agitated will vary depending on a variety of factors such as the velocity of agitation, the size of the housing, the number, size and shape of the test strip(s) in the housing, etc. For example, in those embodiments in which the housing has a length L_{H} ranging from about 30 mm to about 60 mm, a width W_{H} ranging from about 15 mm to about 35 mm and a diameter D_{H} ranging from about 10 mm to about 30 mm and in which is contained from about 1 to about 25 test strips, each test strip having a length L_{T} ranging from about 27mm, a width W_{T} ranging from 6 mm and a thickness T_{T} ranging from about 0.7 mm, the housing is typically agitated in a rotational, angular or pendular manner at least one time (as illustrated in the steps shown in Figures 12A-12C), where the housing may be agitated in a rotational, angular or pendular manner at least two times (as illustrated in the steps shown in Figures 12A-12F) or more.

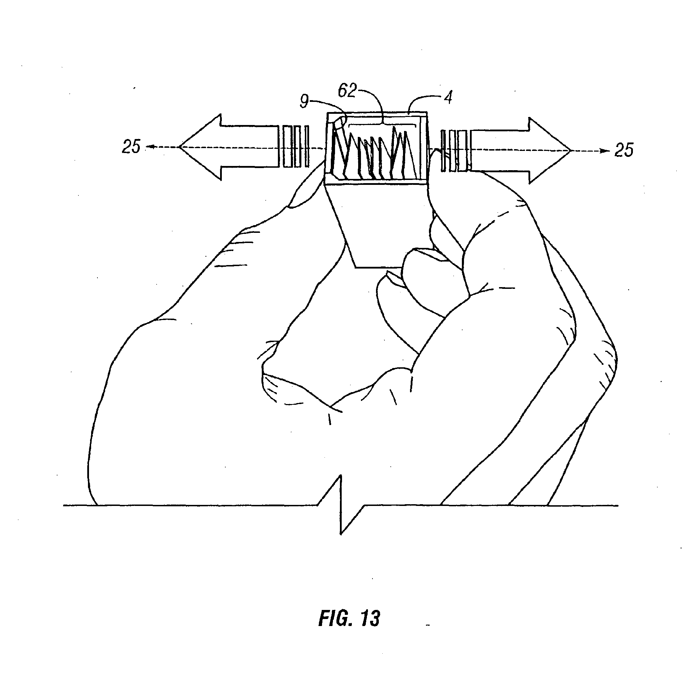

The inventors have discovered the surprising result that if the subject housing is agitated, for example as described above, the at least one test strip contained therein will be caused to be positioned in an appropriate orientation relative to the direction of agitation. By appropriate orientation is meant that the broad surfaces or faces of each test strip contained in the housing (for example broad surfaces 200 and 202, as described above with respect to electrochemical test strip 62) will be substantially perpendicular to the plane or direction of the movement or agitation, as shown in Figure 13. Figure 13 shows a cut-away, top view, such as taken along lines A-A of Figure 6A, into the interior of housing 3 having a plurality of test strips 62 contained therein. Thus, when the housing is agitated, for example along plane 25, the broad surfaces or faces 200 and 202 of each of the test strips 62 contained in the housing align substantially perpendicular to plane 25. Furthermore, because at least one planar surface 9 is positioned adjacent slit 10 such that the longitudinal axis 40 of slit 10 is substantially parallel to the at least one planar surface and the width of the planar surface 9 is at least as wide as the length of the slit 10, upon appropriate agitation a test strip will engage planar surface 9 and be appropriately aligned relative to slit 10.

Accordingly, if slit 10 is positioned so that its longitudinal axis 40 (see Figure 6A) is perpendicular to the direction or plane of agitation, upon agitation of the housing, if the housing is agitated or moved in a direction that is substantially perpendicular to longitudinal axis 40 of slit 10, test strips 62 therein will be appropriately orientated such that broad surfaces or faces 200 and 202 of each of the test strips in housing 3 will always substantially face the at least one planar surface 9. As such, at least one test strip is caused to contact the at least one planar surface 9 that is aligned with and adjacent to slit 10, which in turn causes a test strip to be aligned with slit 10 so that the test strips 62 in the housing will fall into slit 10 one at a time and exit the housing.

Figure 14 shows a single test strip 62a exiting housing 3 while the other test strips remain inside housing 3. As shown, exiting test strip 62a comes to rest on test strip stop 35. In this manner, a portion of the exiting test strip is positioned inside the housing and a portion is positioned outside the housing as test strip 62a is prevented from completely exiting the housing due to the test strip stop. Figure 15 shows a view taken through line B-B of Figure 14. As shown in Figure 15, usually a greater portion or a majority of the test strip 62a, usually a substantial portion or greater than about 50% of the length of test strip 62a, is positioned outside the housing, while the remaining portion is positioned inside the housing such that a portion 63 is positioned above the inside surface of bottom wall 7 housing. In this manner, additional test strips are prevented from entering slit 10 behind or after test strip 62a. Usually the portion of the test strip 62a that is positioned outside the housing is the portion where sample is applied.

Once a test strip is dispensed through slit 10, an individual may choose to leave test strip 62a partially protruding from housing 3 and apply sample thereto in those instances where the sample application portion of test strip 62a is protruding from the housing, i.e., the sample application is accessible. In such a case, it is obvious that test strip 62a is oriented or held in such a manner that sample may be easily applied thereto while partially protruding from the housing and held by the test strip stop, i.e., the sample application area or portion of the test strip is not substantially obscured. Alternatively, an individual may choose to remove test strip 62a from the housing before sample is applied thereto. In this case, an individual simply pulls or tugs test strip 62a out of slit 10 and away from the housing, e.g., with fingers, as shown in Figure 16.

Once a test strip has been removed from the housing, whether done before or after sample application, if applicable, the housing is then re-sealed, either by covering a portion of the housing with a cap 20, if used, and/or by moving test strip stop 35 back to a second position.

The subject methods also include reloading the housing with one or more test strips. As such, a portion of housing 3 is opened and one or more test strips are inserted therein. For example, a cap may be removed to expose, for example, an opening in first side 4 (not shown) through which test strips may be passed to the interior of housing 3. Once filled with one or more test strips, cap 20 may be positioned to once again cover first side 20. In certain embodiments, during the reloading process, more or new moisture absorbent reagents or components such as desiccant material, silica gel and the like, are positioned in the housing, where previously positioned moisture absorbing materials may be removed.

### KITS

Finally, kits for practicing the subject methods are provided. The subject kits at least include one or more test strip container devices of the subject invention. Oftentimes, a plurality of subject devices is included. The subject kits may also include one or more test strips, usually a plurality of test strips, such as the type described above, e.g., electrochemical and colorimetric test strips. The kits may further include a meter for automatically determining the presence and/or concentration of at least one analyte in a physiological sample applied to a test strip. The subject kits may further include an element for obtaining a physiological sample. For example, where the physiological sample is blood, the subject kits may further include an element for obtaining a blood sample, such as a lance for sticking a finger, a lance actuation means, and the like. In addition, the subject kits may include a control solution or standard, e.g., a control solution that has a known analyte concentration such as a known glucose concentration. Also included may be instructions for using the subject devices for dispensing a test strip and may also include instructions for determining the presence and/or concentration of at least one analyte in a physiological sample applied to a test strip. The instructions may be printed on a substrate, such as paper or plastic, etc. As such, the instructions may be present in the kits as a package insert, in the labeling of the container of the kit or components thereof (i.e., associated with the packaging or sub-packaging) etc. In other embodiments, the instructions are present as an electronic storage data file present on a suitable computer readable storage medium, e.g., CD-ROM, diskette, etc.

It is evident from the above description and discussion that the above described invention provides a simple, quick and convenient way to dispense test strips. The above described invention provides a number of advantages, including, but not limited to, ease and low cost manufacture, minimal components, portability and ease of use, particularly for visually and dextrally impaired individuals. As such, the subject invention represents a significant contribution to the art.

The subject invention is shown and described herein in what is considered to be the most practical, and preferred embodiments. It is recognized, however, that departures may be made therefrom, which are within the scope of the invention as claimed, and that obvious modifications will occur to one skilled in the art upon reading this disclosure.

The specific devices and methods disclosed are considered to be illustrative and not restrictive. Modifications that come within the meaning and range of equivalents of the disclosed concepts, and that would readily occur to one skilled in the relevant art, within the scope of the appended claims, may be made.

## Claims

1. A device (1) for containing at least one test strip (62) and dispensing a single test strip at a time, said device comprising:
a housing (2) comprising:
(i) a slit (10) configured to be permissive of a single test strip at a time; and
(ii) at least one planar surface within said housing configured to align a single test strip with said slit when said device is appropriately agitated.

2. The device according to claim 1, wherein said slit has a width greater than the thickness of a single test strip and less than the thickness of two test strips and a length greater than the width of a single test strip and less than the width of two test strips.

3. The device according to claims 1 or 2, wherein said device further comprises a test strip stop (16) configured for holding a single test strip positioned in said slit.

4. The device according to any of the preceding claims, further comprising at least one sloped surface adjacent said slit for directing said at least one test strip towards said slit.

5. The device according to any of the preceding claims, wherein said device comprises at least one cap (20) for sealing said housing.

6. A method for containing at least one test strip (62) and dispensing a single test strip at a time, said method comprising:
(a) providing a device (1) according to any of the preceding claims; and
(b) agitating said device in a manner that causes said at least one test strip to be positioned in an appropriate orientation relative to said slit (10), wherein a single test strip is caused to exit said housing through said slit as a result of said agitation.

7. The method according to claim 6, further comprising applying a sample to said exited test strip.

8. The method according to claim 7, further comprising determining the concentration of at least one analyte in said sample.

9. The method according to claim 8, further comprising inserting said exited test strip in a meter for automatically determining the concentration of said at least one analyte.

10. A kit for containing at least one test strip (62) and dispensing a single test strip at a time, said kit comprising:
(a) at least one device (1) according to claims 1 to 5; and
(b) a substrate comprising instruction for using said at least one device.

## Patentansprüche

1. Vorrichtung (1) zum Halten wenigstens eines Teststreifens (62) und Ausgeben jeweils eines einzelnen Teststreifens, wobei die Vorrichtung aufweist:
ein Gehäuse (2), welches aufweist:
(i) einen Schlitz (10), der so gestaltet ist, daß er jeweils einen einzelnen Teststreifen zuläßt; und
(ii) wenigstens eine planare Fläche innerhalb des Gehäuses, die so ausgestaltet ist, daß sie einen einzelnen Teststreifen mit dem Schlitz ausrichtet, wenn die Vorrichtung in geeigneter Weise geschüttelt wird.

2. Vorrichtung nach Anspruch 1, bei der der Schlitz eine Breite hat, die größer ist als die Dicke eines einzelnen Teststreifens und kleiner ist als die Dicke zweier Teststreifen und eine Länge, die größer ist als die Breite eines einzelnen Teststreifens und kleiner als die Breite von zwei Teststreifen ist.

3. Vorrichtung nach Anspruch 1 oder 2, bei der die Vorrichtung weiter einen Teststreifen-Stop (16) aufweist, der so gestaltet ist, daß er einen einzelnen Teststreifen hält, welcher in dem Schlitz positioniert ist.

4. Vorrichtung nach einem der vorangehenden Ansprüche, weiter mit wenigstens einer schräg verlaufenden Fläche benachbart dem Schlitz zum Führen des wenigstens einen Teststreifens in Richtung auf den Schlitz.

5. Vorrichtung nach einem der vorangehenden Ansprüche, bei der die Vorrichtung wenigstens eine Kappe (20) zum Abdichten des Gehäuses aufweist.

6. Verfahren zum Halten wenigstens eines Teststreifens (62) und zum Ausgeben jeweils eines einzelnen Teststreifens, wobei das Verfahren aufweist:
(a) Bereitstellen einer Vorrichtung (1) nach einem der vorangehenden Ansprüche; und
(b) Schütteln der Vorrichtung in einer Weise, die bewirkt, daß der wenigstens eine Teststreifen in einer geeigneten Ausrichtung relativ zu dem Schlitz (10) positioniert wird, wodurch bewirkt wird, daß ein einzelner Teststreifen aus dem Gehäuse durch den Schlitz als ein Ergebnis des Schüttelns austritt.

7. Verfahren nach Anspruch 6, weiter mit dem Aufbringen einer Probe auf den herausgetretenen Teststreifen.

8. Verfahren nach Anspruch 7, weiter mit dem Bestimmen der Konzentration wenigstens eines Analyten in der Probe.

9. Verfahren nach Anspruch 8, weiter mit dem Einführen des ausgetretenen Teststreifens in ein Meßgerät zum automatischen Bestimmen der Konzentration des wenigstens einen Analyten.

10. Ausstattungsset zum Halten wenigstens eines Teststreifens (62) und zum Ausgeben jeweils eines einzelnen Teststreifens, wobei das Ausstattungsset aufweist:
(a) wenigstens eine Vorrichtung (1) nach den Ansprüchen 1 bis 5; und
(b) ein Substrat mit Instruktionen zum Verwenden der wenigstens einen Vorrichtung.

## Revendications

1. Dispositif (1) destiné à contenir au moins une bandelette de test (62) et à distribuer une seule bandelette de test à la fois, ledit dispositif comprenant :
un logement (2) comprenant :
(i) une fente (10) configurée afin de permettre une seule bandelette de test à la fois ;
et
(ii) au moins une surface planaire dans ledit logement configurée afin d'aligner une seule bandelette de test avec ladite fente lorsque ledit dispositif est correctement agité.

2. Dispositif selon la revendication 1, dans lequel ladite fente possède une largeur supérieure à l'épaisseur d'une seule bandelette de test et inférieure à l'épaisseur de deux bandelettes de test et une longueur supérieure à la largeur d'une seule bandelette de test et inférieure à la largeur de deux bandelettes de test.

3. Dispositif selon les revendications 1 ou 2, dans lequel ledit dispositif comprend en outre une butée de bandelette de test (16) configurée afin de maintenir une seule bandelette de test positionnée dans ladite fente.

4. Dispositif selon l'une quelconque des revendications précédentes, comprenant en outre au moins une surface inclinée adjacente à ladite fente afin de diriger ladite au moins une bandelette de test vers ladite fente.

5. Dispositif selon l'une quelconque des revendications précédentes, dans lequel ledit dispositif comprend au moins un embout (20) destiné à fermer ledit logement.

6. Procédé destiné à contenir au moins une bandelette de test (62) et à distribuer une seule bandelette de test à la fois, ledit procédé comprenant :
(a) le fait de prévoir un dispositif (1) selon l'une quelconque des revendications précédentes ; et
(b) l'agitation dudit dispositif d'une manière qui provoque le fait que ladite bandelette de test au moins soit positionnée selon une orientation appropriée par rapport à ladite fente (10), où une seule bandelette de test sort dudit logement par ladite fente à la suite de ladite agitation.

7. Procédé selon la revendication 6, comprenant en outre l'application d'un échantillon à ladite bandelette de test sortie.

8. Procédé selon la revendication 7, comprenant en outre la détermination de la concentration d'au moins une substance à analyser dans ledit échantillon.

9. Procédé selon la revendication 8, comprenant en outre l'insertion de ladite bandelette de test sortie dans un dispositif de mesure afin de déterminer automatiquement la concentration de ladite substance à analyser au moins.

10. Kit destiné à contenir au moins une bandelette de test (62) et à distribuer une seule bandelette de test à la fois, ledit kit comprenant :
(a) au moins un dispositif (1) selon les revendications 1 à 5 ; et
(b) un substrat comprenant une instruction d'utilisation dudit dispositif au moins.
